# EUROPEAN PATENT SPECIFICATION

(11) **EP 2 411 027 B1**
(45) Date of publication and mention of the grant of the patent: **19.11.2014**
(21) Application number: 10712041.2
(22) Date of filing: 24.03.2010
(51) Int. Cl.: A61K 35/74, A61K 8/99, A61Q 19/06, A61K 31/702, C12R 1/225, A61P 3/04, A61P 3/08

(54) **USE OF A PROBIOTIC TO REGULATE BODY WEIGHT**
VERWENDUNG EINES PROBIOTIKUMS ZUR REGULIERUNG DES KÖRPERGEWICHTS
UTILISATION D'UN PROBIOTIQUE POUR RÉGULER LE POIDS D'UN CORPS

(30) Priority: 25.03.2009 EP 09156109
(43) Date of publication of application: 01.02.2012
(73) Proprietor: Chr. Hansen A/S, 2970 Hoersholm (DK)
(72) Inventor: LESER, Thomas Dyrmann, DK-1870 Frederiksberg (DK); GUNNARSSON, Thomas, S-211 50 Malmoe (SE); KILDSGAARD, Jens, DK-2840 Holte (DK); PEDERSEN, Janni Wandahl, DK-3450 Alleroed (DK); FLAMBARD, Bénédicte, DK-2000 Frederiksberg (DK)
(86) International application number: PCT/EP2010/053826
(87) International publication number: WO 2010/108950

(56) References cited:
- WO-A-2007/043933
- WO-A-2007/085970
- CANI P D ET AL: "Changes in gut microbiota control inflammation in obese mice through a mechanism involving GLP-2-driven improvement of gut permeability." GUT AUG 2009, vol. 58, no. 8, 24 February 2009 (2009-02-24), pages 1091-1103, XP002541001 ISSN: 1468-3288
- TENNYSON C A ET AL: "Microecology, obesity, and probiotics" CURRENT OPINION IN ENDOCRINOLOGY, DIABETES AND OBESITY, LIPPINCOTT WILLIAMS & WILKINS, LTD, GB, vol. 15, no. 5, 1 January 2008 (2008-01-01), pages 422-427, XP009121254 ISSN: 1752-296X
- TANIDA M ET AL: "High-fat diet-induced obesity is attenuated by probiotic strain Lactobacillus paracasei ST11 (NCC2461) in rats" OBESITY RESEARCH & CLINICAL PRACTICE,, vol. 2, no. 3, 1 September 2008 (2008-09-01), pages 159-169, XP024531134 ISSN: 1871-403X [retrieved on 2008-05-27]
- DE MORENO DE LEBLANC A ET AL: "Oral administration of L. casei CRL 431 increases immunity in bronchus and mammary glands" EUROPEAN JOURNAL OF INFLAMMATION, BIOMED CENTRAL, IT, vol. 3, no. 1, 1 January 2005 (2005-01-01) , pages 25-28, XP009109241 ISSN: 1721-727X
- AGUERO G ET AL: "Beneficial immunomodulatory activity of Lactobacillus casei in malnourished mice pneumonia: effect on inflammation and coagulation" NUTRITION, ELSEVIER INC, US, vol. 22, no. 7-8, 1 July 2006 (2006-07-01) , pages 810-819, XP025114640 ISSN: 0899-9007 [retrieved on 2006-07-01]

## Description

### FIELD OF THE INVENTION

The invention relates to the strain *Lactobacillus paracasei* subsp. *paracasei* having accession number ATCC 55544 that modulates expression of satiety markers (e.g. factors coded by the GCG gene) in the intestine while at the same time increasing fat oxidation and reduction of fat deposition in muscle tissue. Consumption of the probiotic strain may thus help to promote an optimal body weight of a mammal. The invention further relates to a composition comprising the strain for use in the prevention and/or treatment of obesity, type 2 diabetes mellitus and polycystic ovary syndrome (PCOS) as well as cosmetic methods for reducing body weight

### BACKGROUND OF THE INVENTION

### Body weight management

The healthy, well functioning body of a mammal (including humans) is characterized by an optimal weight. The specific optimal weight varies widely according to species, gender, age, type of body stature, level of physical activity etc. of the individual mammal. It is however clear that an optimal body weight range can be established for any individual mammal, and that extensive over- as well as under-weight have drastic negative effects on the health and wellbeing of the individual.

In general, during evolution the mammals have adapted to a situation of scarce food resources and famine, and complex mechanisms have evolved to cope with this situation, one being the hunger signaling, which completely changes the behavior pattern of most mammals, and also the mammals' ability to store large energy resources in the form of body fat.

The maintenance of the optimal body weight is complex and multifactorial (NIH 1998). It involves a multitude of signaling pathways and metabolic processes as well as a spectrum of genetic and environmental factors. Present clinical evidence indicates that a multi-faceted intervention involving several signaling pathways and metabolic processes is required to obtain an effect full treatment of obesity.

Within the last decade it has become increasingly clear that the healthy mammalian body also has developed a number of intricate mechanisms that regulate the feed intake during periods of surplus by regulating our satiety. Many of these mechanisms seem to involve specific responses to certain components in the food, and the molecular details of more satiety signaling pathways have been revealed and have shown to involve specific signaling molecules/hormones. Depending on their specific levels (presence or absence) such specific satiety regulating signaling molecules/hormones may signal either satiety or hunger. Collectively they are here referred to as "satiety markers".

### Satiety markers

### Proglucagon and derived peptide hormones.

The gene coding for the glucagon precursor, proglucagon, is expressed in the brain, pancreas, and in the small and large intestine. The gene is also referred to as the "GCG gene" or simple "GCG" (Ensembl: ENSG00000115263).".

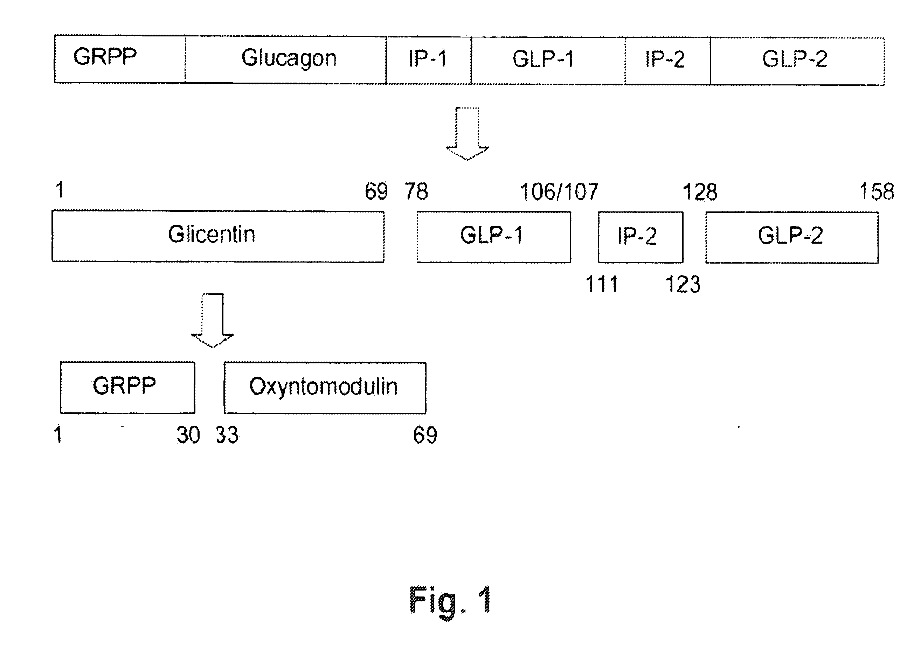

In L-cells, located primarily in the epithelium of the distal small intestine and in the colon, the polypeptide proglucagon is cleaved to GLP-1 (glucagon-like peptide 1), GLP-2 (glucagon-like peptide 2), oxyntomodulin, glicentin and IP-2 (intervening peptide 2) (see figure 1). While GLP-1 and GLP-2 exert well-defined actions through known receptors, the biological function of glicentin and IP-2 remains less well characterized and no receptors have been identified. The function of oxyntomodulin has been deduced from animal experiments and human trials however, no receptor has yet been identified.

### Physiology of glucagon-like peptide-1 (GLP-1)

In L cells of the intestine, GLP-1 (formerly called insulinotropin) is produced as a 39-amino acid peptide which is stored intracellularly. GLP-1 is released into the circulation in response to nutrient ingestion and has a number of physiological effects (1).

Several studies have demonstrated that the observed improvements in glycemic control following long-term peripheral administration of GLP-1 or pharmacological GLP-1 analogues in animal models and in patients with type 2 diabetes (T2DM) are associated with significant reductions in body weight (2), indicating that GLP-1 may play a role in the regulation of energy balance. In a recent meta-analysis it was concluded that GLP-1 reduces appetite and caloric intake, the latter by an average of 11.7% acutely. The reduction is similar in lean and obese subjects and is achieved without adverse effects (3). It is well-known that GLP-1 inhibits gastric emptying. Reduced gastric emptying generate prolonged stretching of the stomach after food intake. Mechanoreceptors, located in the stomach, quantify the stretch of the stomach and signal satiety to the brain. The satiating effect of GLP-1 may also be caused by GLP-1 acting directly in the brain, as GLP-1 immunoreactive neurons are found in large quantities in the central nerve system (CNS) areas involved in appetite regulation (4-6). Several studies have confirmed the presence of GLP-1 receptors in areas of the brain important to appetite regulation, supporting the notion that GLP-1 is involved in central appetite control (7;8).

### Physiology of glucagon-like peptide-2 (GLP-2)

As for GLP-1, GLP-2 is produced by posttranslational processing of the polypeptide proglucagon in L cells of the intestine. GLP-2 is as a 33-amino acid peptide which is co-secreted with GLP-1 in response to ingestion of nutrients, especially lipids and carbohydrates (9).

GLP-2 has been proposed to act as a regulator of food intake. When rats received in-tracerebroventricular administration of GLP-2 food intake was inhibited (10). In contrast to GLP-1, central administration of GLP-2 does not inhibit water intake and does not cause conditioned taste aversion.

In mice, subcutaneous GLP-2 injections enhances intestinal epithelial barrier function affecting both the paracellular and transcellular pathways (11). An improved gastro-intestinal barrier function is associated with a reduction in the flux of lipopolysaccharides (LPS) from the gut lumen into the circulating system. Even moderately increased levels of LPS have recently been shown to induce adipose weight gain similar to what is obtained by a high-fat diet in rodents (12).

### Physiology of oxyntomodulin

Oxyntomodulin (also referred to as glucagons-37, glicentin-(33-69), and in older references as bioactive enteroglucagon) is produced by posttranslational processing of the polypeptide proglucagon in L cells of the intestine.

Oxyntomodulin shares many properties with GLP-1. Consequently, exogenously administered oxyntomodulin can acutely ameliorate glucose intolerance in diet-induced obese mice and this is likely due to stimulation of glucose-induced insulin secretion (13). Furthermore, oxyntomodulin can delay gastric emptying and reduce gastric acid secretion (14). Importantly, the administration of exogenous oxyntomodulin results in both short-term effects on feeding and more long-term effects on body weight gain in both rodents and human subjects. Oxyntomodulin has been shown to acutely decrease the sensation of hunger and inhibit caloric intake in normal healthy subjects. In the same study, oxyntomodulin administration reduced circulating ghrelin levels by approximately 44% (15). It is possible that the suppressive effect on feeding is mediated via the reduction of ghrelin, a hunger hormone produced by endocrine cells lining the stomach. It has also been suggested that oxyntomodulin may increase energy expenditure (16), which together with reduced energy intake may result in a negative energy balance leading to weight loss. Indeed, seven-day administration (i.p.) of oxyntomodulin reduced the rate of body weight gain and adiposity in rats (17). Similarly, four weeks oxyntomodulin treatment (by subcutaneous injection) resulted in 2.3 kg weight loss (compared to 0.5 kg for the control group) in overweight and obese human subjects (18). These studies clearly indicate that oxyntomodulin may be involved in the regulation of food intake and body weight gain.

### Physiology of glicentin

Glicentin (also referred to as enteroglucagon, glucagon-69, or gut-type glucagon) corresponds to amino acids 1-69 of preproglucagon. The sequence also comprises the sequence of oxyntomodulin (glicentin-(33-69)). Glicentin 1-30 corresponds to GRPP (glicentin-related pancreatic peptide) (19).

Glicentin is produced in the intestinal L cells and is secreted during digestion. Glicentin slows down gastric emptying and can switch off the duodenojejunal fed motor pattern. Tomita *et al.* (2005) (20) have reported that glicentin plays an important role in the regulating inhibition of the contraction reaction in normal human jejunum via non-adrenergic noncholinergic nerves, and has a direct action on the jejunal muscle receptor. In contrast, Ayachi *et al.* (2005) (21) have shown that glicentin contributes to contraction of smooth muscle cells isolated from human colon. Exendin-(3-39), described as a GLP-1 receptor antagonist, inhibited contraction due to glicentin, suggesting that glicentin may act through the GLP-1 receptor Ayachi *et al.* (2005) (21).

### The biology and physiology of peptide YY.

Peptide YY (also known as PYY, Peptide Tyrosine Tyrosine, or Pancreatic Peptide YY₃₋₃₆), Ensembl: ENSG00000131096, is encoded by the human chromosome 17 band q21.1. There are two major forms of Peptide YY: PYY₁₋₃₆ and PYY₃₋₃₆. Peptide YY₃₋₃₆ (PYY) is a linear polypeptide consisting of 34 amino acids with structural homology to NPY and pancreatic polypeptide Peptide YY is related to the pancreatic peptide family by having 18 of its 34 amino acids locate in the same positions as pancreatic peptide(22). The most common form of circulating PYY is PYY₃₋₃₆ which binds to the Y2 receptor (Y2R)(23)..

PYY is found in L cells in the mucosa of gastrointestinal tract, especially in ileum and colon. A small amount of PYY, about 1-10 percent, is produced in the esophagus, stomach, duodenum and jejunum(24). The plasma PYY concentration increases postprandially (after food ingestion) and decreases by fasting(23).

PYY exerts its action through NPY receptors, inhibits gastric motility and increases water and electrolyte absorption in the colon(25). PYY may also suppress pancreatic secretion. It is secreted by the enteroendocrine cells in the ileum and colon in response to a meal, and has been shown to reduce appetite. PYY works by slowing the gastric emptying; hence, it increases efficiency of digestion and nutrient absorption after a meal.

Several studies have shown that acute peripheral administration of PYY₃₋₃₆ inhibits feeding of rodents and primates. Studies on Y2R-knockout mice have indicated no anorectic (losing appetite) effect on Y2R-knockot mice. These findings suggest that PYY₃₋₃₆ has anorectic effect which is mediated by Y2R. PYY-deficient female mice have increased body weight and fat mass. PYY-deficient mice, on the other hand, are resistant to obesity but have higher fat mass and lower glucose tolerance when fed with high-fat diet, compare to control mice. Thus PYY also plays very important role in energy homeostasis by balancing the food intake(23).

In human volunteers receiving artificial infusions of PYY at normal post-eating concentrations, food intake was reduced by a third for a day. The researchers of this study also investigated the hunger levels of the test group both during and after transfusions of the hormone. The group receiving PYY reported up to a 40% reduction in perceived levels of hunger over a period of 12 hours following infusion(26).

These data suggest the PYY may be useful as a potential therapy for obesity or at least for reducing appetite in individuals on a weight-reduction diet.

### The biology and physiology of apolipoprotein A-IV

Apolipoprotein A-IV is encoded by APOA4 (alias Apo-AIV or ApoA-IV) with gene ID:, Ensembl: ENSG00000110244, is encoded by the human chromosome 11 band q23. APOA4 contains 3 exons separated by two introns. The primary translation product is a 396-residue preprotein which after proteolytic processing is secreted in association with chylomicron particles.

Apolipoprotein A-IV (apoA-IV) acts as a satiety factor. ApoA-IV is a 46,000-Da glycoprotein synthesized by the human intestine. In rodents, both the small intestine and liver secrete apoA-IV, but the small intestine is the major organ responsible for circulating apoA-IV(27). There is now solid evidence that the hypothalamus, especially the arcuate nucleus, is another active site of apoA-IV expression(28). Intestinal apoA-IV synthesis is markedly stimulated by fat absorption and does not appear to be mediated by the uptake or re-esterification of fatty acids to form triglycerides. Rather, the local formation of chylomicrons acts as a signal for the induction of intestinal apoA-IV synthesis. Intestinal apoA-IV synthesis is also enhanced by a factor from the ileum, probably peptide tyrosine-tyrosine (PYY)(29). The inhibition of food intake by apoA-IV is mediated centrally(30). The stimulation of intestinal synthesis and secretion of apoA-IV by lipid absorption is rapid; thus apoA-IV likely plays a role in the short-term regulation of food intake. Other evidence suggests that apoA-IV may also be involved in the long-term regulation of food intake and body weight, as it is regulated by both leptin and insulin(28). Chronic ingestion of a high-fat diet blunts the intestinal as well as the hypothalamic apoA-IV response to lipid feeding(29).

Thus, peptide signaling molecules (hormones) such as GLP-1, GLP-2, Oxyntomodulin, Glicentin and PYY as well as proteins such as apolipoprotein A-IV, are involved in the signaling of and the response to either satiety or hunger and can accordingly be referred to as "satiety markers".

### Fatty acid metabolism

### The biology and physiology of Stearoyl-CoA desaturase-1 (SCD1)

Stearoyl-CoA desaturase (SCD; EC 1.14.99.5) is an iron-containing enzyme that catalyzes a rate-limiting step in the synthesis of unsaturated fatty acids. SCD-1 is encoded by a gene on human chromosome 10q24.31 with Entrez gene ID: 6319.

Stearoyl-CoA desaturase-1 (SCD1) determines fatty acid partitioning into lipogenesis or fatty acid β-oxidation in muscle tissue. Upregulation of SCD1 is seen in obese individuals and results in accumulation of intramyocellular triacylglycerol (IMTG). Human obesity is associated with abnormal accumulation of neutral lipids within skeletal myofibers. This phenomenon occurs in concert with reduced insulin stimulated glucose transport and impaired insulin signal transduction. Pharmacological and genetic manipulations that deplete IMTG restore insulin sensitivity. Hulver et *al.* (2005) (31) have identified a linear relationship befurther, "that pharmacological targeting of muscle SCD1 and/or its upstream regulators could provide new opportunities for preventing and/or treating obesity and its related comorbidities (31).

### Probiotics

Probiotic microorganisms have been defined as "Live microorganisms which when administered in adequate amounts confer a health benefit on the host" (FAO/WHO 2001).

It has been described that certain probiotic bacterial strains may have the ability to modulate the level of specific satiety markers.

WO 2007/085970 A2 (DANISCO A/S) describes specific strains of *Lactobacillus acidophilus, Lactobacillus curvatus, Lactobacillus salivarius* and *Bifidobacterium lactis* that are capable of modulating satiety signaling, specifically mentioning the group of satiety markers consisting of PYY, CCK, Ghrelin, Leptin, GLP-I, orexins, orexigenic hypothalamic neuropeptide Y, acetic acid, amylin and oxyntomodulin.

*Lactobacillus casei* and *Lactobacillus paracasei* are only disclosed in very general terms at page 21, lines 1-2 and 9. The specific strain *Lactobacillus paracasei* subsp. *paracasei* CRL431 having accession number ATCC 55544 is not mentioned. The examples are based upon studies of Caco-2 cells treated with *Lactobacillus acidophilus* PTA-4797, *B. lactis* 420, *B. lactis* HN019, *L. salivarius* Ls-33, *L. acidophilus* NCFM and *L. curvatus* 853. Further a rat model and clinical trials with human are proposed but no results are provided.

WO 2007/085970 fails to disclose or point to *Lactobacillus paracasei* subsp. *paracasei* CRL431 having accession number ATCC 55544 for use in the prevention and/or treatment of obesity, type 2 diabetes mellitus and polycystic ovary syndrome (PCOS) or in cosmetic methods for reducing body weight.

WO 00238165A1 (PROBI) describes specific *Lactobacillus plantarum* strains that modulate the levels of insulin and leptin.

US 2004/0048356 A1 (Johansson) describes two specific strains of Lactobacillus plantarum giving rise to increased amounts of propionic acid or acetic acid in the colon, that eventually modify the leptin level and the PPAR gene expression.

Tennyson CA et al., 2008 is a review article. Under the heading "Probiotics and obesity treatment" it is held (page 6, left column, 2^{nd} paragraph) that the literature demonstrating the effects of probiotics on obesity or metabolism is limited. There is no hint or suggestion to use *Lactobacillus paracasei* subsp. *paracasei* CRL431.

Tanida et al., 2008 concludes that the results suggest that *Lactobacillus paracasei* ST11 (NCC2461) has an anti-obese action, and in this mechanism, autonomic nerves may function to facilitate the lipolytic and thermogenic responses via the sympathetic excitation and to suppress the parasympathetic nerve activity in rats.

A thorough review of Tennyson CA et al., 2008 and Tanida et al., 2008 do not provide any suggestion or hint that the specific strain *Lactobacillus paracasei* subsp. *paracasei* CRL431 having accession number ATCC 55544 could be useful in the prevention and/or treatment of obesity, type 2 diabetes mellitus and polycystic ovary syndrome (PCOS) or in cosmetic methods for reducing body weight.

WO2007/043933 discloses use of at least one of *Lactobacillus casei* F19 (LMG P-17806), *Lactobacillus acidophilus* NCFB 1748 or *Bifidobacterium lactis* Bb12 for controlling weight gain etc.

However, neither WO2007/043933 provides any suggestion or hint that the specific strain *Lactobacillus paracasei* subsp. *paracasei* CRL431 having accession number ATCC 55544 could be useful in the prevention and/or treatment of obesity, type 2 diabetes mellitus and polycystic ovary syndrome (PCOS) or in cosmetic methods for reducing body weight.

To the best of our knowledge there is no report of a single class of bacteria that is able to: 1. Modulate all of GLP-1, GLP-2, Oxyntomodulin and Glicentin through activation of the GCG gene in the intestine of an animal, 2. Up-regulate the expression of PYY in the intestine and in cell cultures, 3. Up-regulate the expression of APOA4 in the intestine and 4. Increase fat oxidation on muscle tissue via down-regulation of SCD-1.

### SUMMARY OF THE INVENTION

The present invention rotates to a composition comprising *Lactobacillus paracasei* subsp. *paracasei* having accession number ATCC 55544 for use in the prevention and/or treatment of obesity, type 2 diabetes mellitus and polycystic ovary syndrome (PCOS). Said composition up-regulates expression of satiety markers coded by the GCG gene in the intestine of said mammal. The strain also up-regulates expression of satiety markers coded by the APOA4 gene and/or satiety markers coded by the Peptide YY in the intestine of the mammal. Further, said strain down-regulates expression of the SGD1 gene in skeletal muscles of the mammal.

The proglucagon molecule coded by the GCG gene gives rise to a number of known or putative satiety signaling molecules. Accordingly the *Lactobacillus paracasei* subsp. *paracasei* strain in addition to the modulation of the GCG gene at the mRNA level furthermore modulates the level of one or more of the signaling molecules selected from the group consisting of: GLP-1, GLP-2, Oxyntomodulin, IP-2, GRPP and Glicentin at the protein level, from the modulates the level of PYY and/or apolipoprotein A-IV and down-regulates expression of the SCD1 gene in skeletal muscles of the mammal which is important since the down-modulation of SCD1 is expected to increase fat metabolism and thus augment the efficacy of the *Lactobacillus paracasei* subsp. *paracasei* strain CRL431 for reducing over-weight and/or treating obesity.

A composition comprising the strain may be useful for the preparation of a composition for body weight management of a mammal.

One particularly interesting aspect is the use of a composition comprising the strain for use in the prevention or treatment of obesity. It is well known that obesity (BMI ≥ 30) but also overweight (i.e. BMI 25-30) may have serious medical implications and that both obese and overweight individuals may benefit from a weight reduction. However, even normal or near-normal weight individuals (i.e. BMI 18.5-24.9) who do not suffer under medical implications due to overweight may find it attractive to maintain or strive for an optimal body weight for cosmetic reasons. Thus, one additional aspect of the invention is a cosmetic method for reducing body weight in a non-obese, non-overweight subject having a Body Mass Index (BMI) less than 25, said method comprise providing a composition comprising *Lactobacillus paracasei* subsp. *paracasei* having accession number ATCC 55544.

The composition comprising the strain for use according to the invention may be formulated in both liquid and solid dosage forms. In the latter case, the product may be powdered and formed into tablets, granules or capsules or simply mixed with other food ingredients to form a functional food.

### DEFINITIONS

Prior to a discussion of the detailed embodiments of the invention is provided a definition of specific terms related to the main aspects of the invention.

As used herein the term "BMI" designates body mass index. BMI is a measure of the weight of a person scaled according to height. It is defined as the individual's body weight divided by the square of their height (weight measured in kilograms, height in meters). The formula universally used in medicine produce a unit of measure of kg/m². According to the US Department of Health & Human Services a BMI below 18.5 indicates underweight, 18.5-24.9 normal weight, 25-29.9 overweight and a BMI of 30 and above indicates obesity. It should be noted that not only obesity but also overweight (BMI 25-29.9) increases the risk of mortality in adults (Neovius et al (2009) BMJ 338, b496). Accordingly overweight is not only of relevance because of cosmetic indications but also for its medical implications.

By the expression "risk factors involved in overweight and/or obesity" is referred to one or more of the many biochemical factors that are negatively involved in the development of overweight and/obesity. One particular interesting group of such risk factors are the so-called "satiety markers". By the term "satiety markers" is referred to peptides or hormones (including the genes coding for said peptides/hormones) that are involved in the signaling of and the response to satiety which upon increased levels of the factors provide an animal with the feeling of satiety and thereby lead to a reduced feed intake. GLP-1, GLP-2, Oxyntomodulin, and Glicentin, and PYY are examples of such peptide signaling molecules.

The gene referred to as the "GCG gene" or simply "GCG" is the gene coding for the glucagon precursor, proglucagon, the GCG gene is also referred to as the "proglucagon gene".

By the expression "probiotics or probioticum" is referred to a composition which comprises probiotic microorganisms. Probiotic bacteria are defined as microbial cells that have a beneficial effect on the health and well-being of the host. Probiotic microorganisms have been defined as "Live microorganisms which when administered in adequate amounts confer a health benefit on the host" (FAO/WHO 2001).

By the expression "prebiotic" is referred to a composition or a component of a composition which increases the number of probiotic bacteria in the intestine. Thus, prebiotics refer to any non-viable food component that is specifically fermented in the colon by indigenous bacteria thought to be of positive value, e.g. bifidobacteria, lactobacilli. The combined administration of a probiotic strain with one or more prebiotic compounds may enhance the growth of the administered probiotic *in vivo* resulting in a more pronounced health benefit, and is termed synbiotic.

Embodiments of the present invention are described below, by way of examples only.

### DETAILED DISCLOSURE OF THE INVENTION

The invention relates to the use of probiotic bacteria to promote an optimal body weight of a mammal. To the surprise of the inventors, compositions comprising *Lactobacillus paracasei* subsp. *paracasei* strain CRL431 bacteria are able specifically to induce the expression of: 1. The GCG gene in the distal ileum part of the intestine of a mammal (example 1), 2. The APOA4 gene (example 4), 3. GLP1, GLP-2 and PYY secretion in the distal ileum part of the intestine of a mammal (example 2), 4. Secretion of PYY from a human enteroendocrine cell line (example 3), and 5. Decreased expression of SCD-1 in skeletal muscle tissue (example 5). Without wishing to be bound by theory it is perceivable that *Lactobacillus paracasei* subsp. *paracasei* cells can be used for the preparation of a composition for administration to a mammal for modulating expression of the GCG and APOA4 genes gene in the intestine, increasing PYY secretion in the intestine, and increasing fat oxidation in skeletal muscle tissue.

Although the invention is not to be construed as limited by any particular theory, it is thought that due to the fact that maintenance of the optimal body weight involves a multitude of individual signaling pathways and metabolic processes, it follows that a *Lactobacillus paracasei* subsp. *paracasei* strain which up-regulates more satiety-factors will prove to have improved efficacy.

As illustrated in example 1 the probiotic *Lactobacillus paracasei* subsp. *paracasei* strain CRL431 (ATCC 55544) is particularly effective in activating the expression of the GCG gene.

As further illustrated in example 2 - 5 the probiotic *Lactobacillus paracasei* subsp. *paracasei* strain CRL431 (ATCC 55544) also up-regulates expression of the APOA4 gene and the PYY gene, in the intestine, and furthermore down-regulates expression of the SCD1 gene in skeletal muscles of said mammal. Thus this *Lactobacillus paracasei* subsp. *paracasei strain* appears to be unique in that it up-regulates a multitude of satiety-factors and in addition increase fatty acid oxidation in the muscles.

It is contemplated that strains that are directly derived from this probiotic strain are likely to retain their probiotic features including the feature of increasing the expression of genes coding for various satiety-factors and/or lipid metabolism.

The strain *Lactobacillus paracasei* subsp. *paracasei* CRL431 was deposited according to the Budapest Treaty on the International Recognition of the Deposit of Microorganisms for the Purposes of Patent Procedure with the American Tissue type Collection Center, 10801 University Blvd, Manassas, VA 20110, USA on 24 January 1994 under accession number ATCC 55544. The CRL431 strain is commercially available from Chr. Hansen A/S, 10-12 Boege Alle, DK-2970 Hoersholm, Denmark, under the product name Probio-Tec® F-DVS L.casei-431®; Item number 501749, and under the product name Probio-Tec® C-Powder-30, Item number 687018.

In the intestine the proglucagon mRNA gives rise to the production of a number of peptides [ex. 2 expression of biological active protein GLP-1 GLP-2 and PYY increased] also, i.e. GLP-1, GLP-2, Oxyntomodulin, IP-2, GRPP and Glicentin, most of which are known to be or suspected to be signaling molecules involved the signaling of and the response to either satiety or hunger

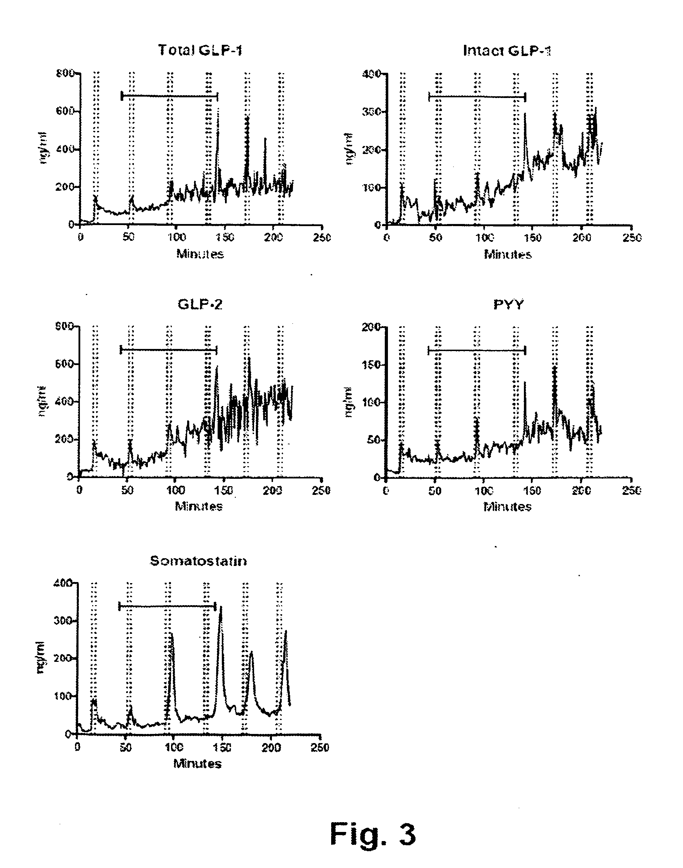

To further substantiate the finding that CRL431 increase the expression of the GCG gene, the gene products, *i.e.* intact GLP-1, GLP-2, Oxyntomodulin and total GLP-1 as well as GLP-2, were measured in the venous effluent of an isolated pig intestine perfused with the CRL431 strain (see example 2). As shown in figure 3, the concentration of intact, biologically active GLP-1 increased by 443% following perfusion of the isolated pig intestine with CRL431. The levels of total GLP-1 and GLP-2 increase by 330% and 460%, respectively, indicating an increased secretion of GCG derived proglucagon hormones. Furthermore, secretion of the satiety inducing hormone PYY was also induced by perfusion with CRL431. As indicated in figure 3, PYY levels increased by 228% after perfusion with CRL431. Therefore one preferred embodiment of the invention is a composition comprising the strain for modulating expression of GLP-1.

As further illustrated in example 3, CRL431 induces the secretion of PYY in cell cultures of a human enteroendocrine cell line (NCI-H716). In this example, CRL431 induce PYY secretion in a dose-dependent manner to reach levels 1131% above background.

Evidence clearly indicates that GLP-1, GLP-2, Oxyntomodulin, Glicentin, and PYY are hormones that are either directly or indirectly (e.g. via increased intestinal epithelial barrier function) able to promote an optimal body weight of a mammal. Particularly the strain for use in the prevention or treatment of obesity is a preferred embodiment of the invention.

In example 4, we show that the probiotic *Lactobacillus paracasei* subsp. *paracasei* strain CRL431 (ATCC 55544) increases the expression of the APOA4 gene in intestinal tissue. It has been shown that the APOA4 gene product, apolipoprotein A-IV is a satiety inducing protein.

Furthermore, as illustrated in example 5 the probiotic *Lactobacillus paracasei* subsp. *paracasei* strain CRL431 (ATCC 55544) decreases the expression of the SCD-1 gene in skeletal muscle tissue. It is contemplated that strains that are directly derived from this probiotic strain are likely to retain their probiotic features including the feature of decreasing the expression of the SCD-1 gene in skeletal muscle tissue.

Obesity is a major risk factor for developing a number of diseases and symptoms. According to The Endocrine Society or The Hormone Foundation (http://www.obesityinamerica.org) overweight and obese people are at an increased risk for developing the following conditions: cardiovascular diseases (e.g. atherosclerosis, hypertension, stroke, congestive heart failure, Angina pectoris), type 2 diabetes mellitus, obesity-related hypoventilation, back and joint problems, non-alcoholic fatty liver disease, gastroesophageal reflux disease, reduced fertility, hypothyroidism, dyslipidemia, hyperinsulinemia, cholecystitis, cholelithiasis, osteoarthritis, gout, sleep apnea and other respiratory problems, polycystic ovary syndrome (PCOS), pregnancy complications, psychological disorders, uric acid nephrolithiasis (kidney stones), stress urinary incontinence and increased incidence of certain cancers (e.g. cancer of the kidney, endometrium, breast, colon and rectum, esophagus, prostate and gall bladder).

Type 1 diabetes (T1DM) is the result of autoimmune destruction of the insulin-producing β-cells in the pancreas. Animal studies indicate that GLP-1 therapy may delay the onset of T1DM by inducing β-cell neogenesis and proliferation (32)22). It has been suggested that this effect is related to anti-inflammatory properties of GLP-1 (33, 23).

Accordingly, important embodiments of the invention are compositions comprising the strain CRL431 having accession number ATCC 55544 for use in the prevention and/or treatment of type 2 diabetes mellitus or polycystic ovary syndrome (PCOS).

When preparing such food or feed products manufacturers usually make use of a so-called starter cultures being cultures used to process food and feed products. Starter cultures are widely used in the diary industry. Typically starter cultures impart specific features to various food or feed products. It is a well established fact that the consistency, texture, body and mouth feel is strongly related to the EPS production of the starter culture used to prepare the food or feed.

By the expression "prebiotic" is referred to a composition or a component of a composition which increases the number of probiotic bacteria in the intestine. Thus, prebiotics refer to any non-viable food component that is specifically fermented in the colon by indigenous bacteria thought to be of positive value, e.g. lactobacilli. The combined administration of a probiotic strain with one or more prebiotic compounds may enhance the growth of the administered probiotic *in vivo* resulting in a more pronounced health benefit. Therefore one further embodiment of the invention is the use of a composition comprising living probiotic bacteria according to the invention in combination with at least one prebiotic. An embodiment wherein the prebiotic is selected from the group: inulin, a transgalacto-oligosaccharide, palatinoseoligosaccharide, soybean oligosaccharide, gentiooligosaccharide, xylooligomers, nondegradable starch, lactosaccharose; lactulose, lactitol, maltitol, FOS (fructo-oligosaccharides), GOS (galacto-oligosaccharides) and polydextrose is especially preferred.

The composition comprising *Lactobacillus paracasei* subsp. *paracasei* CRL431 having accession number ATCC 55544 for use in the prevention and/or treatment of obesity type 2 diabetes mellitus, polycystic ovary syndrome (PCOS).

A cosmetic method for reducing body weight in a non-obese, non-overweight subject having a Body Mass Index (BMI) less than 25, said method comprising providing a composition comprising *Lactobacillus paracasei subsp. paracasei* having accession number ATCC 55644.

A cosmetic method for reducing body weight in a non-obese subject, said method comprise providing a composition comprising *Lactobacillus paracasei subsp. paracasei* having accession number ATCC 55544.

The composition according to the invention, wherein the strain is combined with at least one prebiotic.

A composition according to the above, wherein the at least one strain and/or a fraction and/or metabolite is combined with at least one prebiotic, wherein the at least one prebiotic is selected from the group consisting of: inulin, a transgalacto-oligosaccharide, palantinoseoligosaccharide, soybean oligosaccharide, gentiooligosaccharide, oxylooligomers, nondegradable starch, lactosaccharose; lactulose, lactitol, maltitol, FOS (fructo-oligosaccharides), GOS (galacto-oligosaccharides), and polydextrose.

The invention is further illustrated in the following non-limiting examples and the figures.

### BRIEF DESCRIPTION OF THE DRAWINGS

Figure 1: Schematic representation of the human proglucagon molecule and the proglucagon-derived peptides produced in L-cells of the small and large intestine. The numbers refer to the relative amino acid positions within proglucagon. GRPP: Glicentin-related pancreatic polypeptide. Figure adapted from Wallis *et al.* 2007 (19).

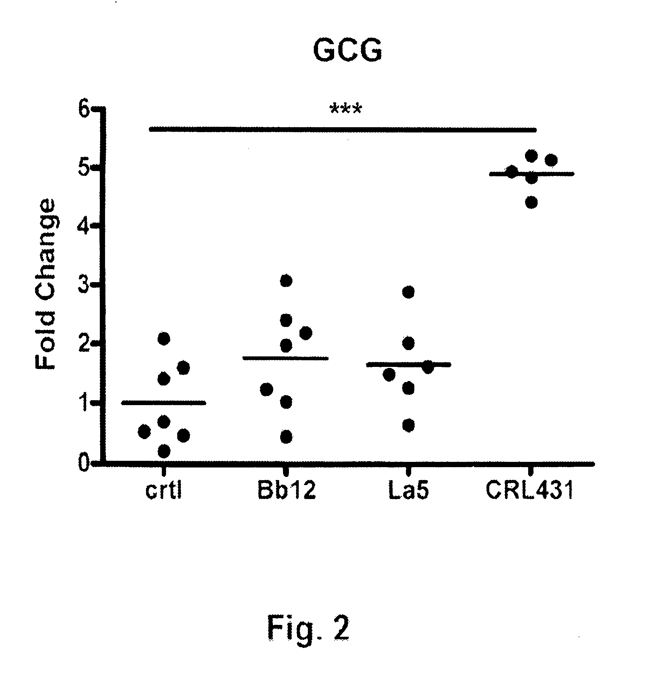

Figure 2: Expression of GCG in porcine ileum. GCG expression was quantified by Q-PCR on RNA extracted from mucosa samples. Each dot represents an individual pig. Average expression is presented as a back line. The average expression of the control group (crtl) was set at 1.0. Bb12: *Bifidobacterium animalis* subsp. *lactis* strain BB-12® (DSM15954); La5: *Lactobacillus acidophilus* strain La5 (DSM13241); CRL431: *Lactobacillus paracasei* subsp. *paracasei* strain CRL431, (ATCC 55544). *** p<0.001 (t-test).
Figure 3: The central ileum of an overnight fasted pig was isolated. The artery, vein, and gut lumen of the isolated ileum were perfused with buffers as described. The perfusion was carried out from time point 0 to 220 minutes. The isolated ileum was stimulated six times with arterial bombesin (indicated as vertical dashed lines). The gut lumen was perfused with CRL431 from time points 39 to 146 minutes (indicated by the horizontal capped line). The venous effluent was collected at 1 minute periods. Intact and total GLP-1, GLP-2, and somatostatin was analyzed in the venous effluent using previously described radioimmunoassays and PYY was analyzed in the venous effluent using ELISA kits from Linco (Millipore) (34).

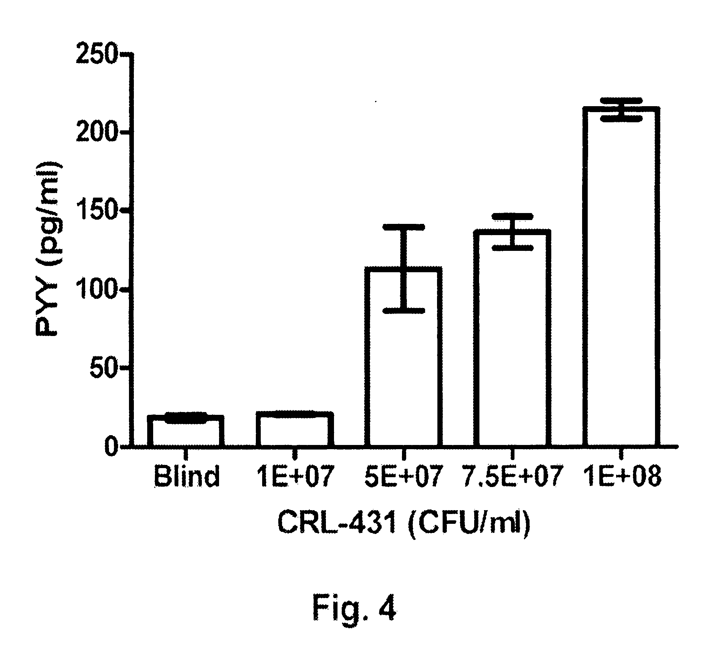

Figure 4: Release of PYY from NCI-H716 enteroendocrine cells stimulated with CRL431 for two hours. NCI-H716 enteroendocrine cells were cultivated as described and CRL-431 co-incubated with the cell culture at indicated concentrations.

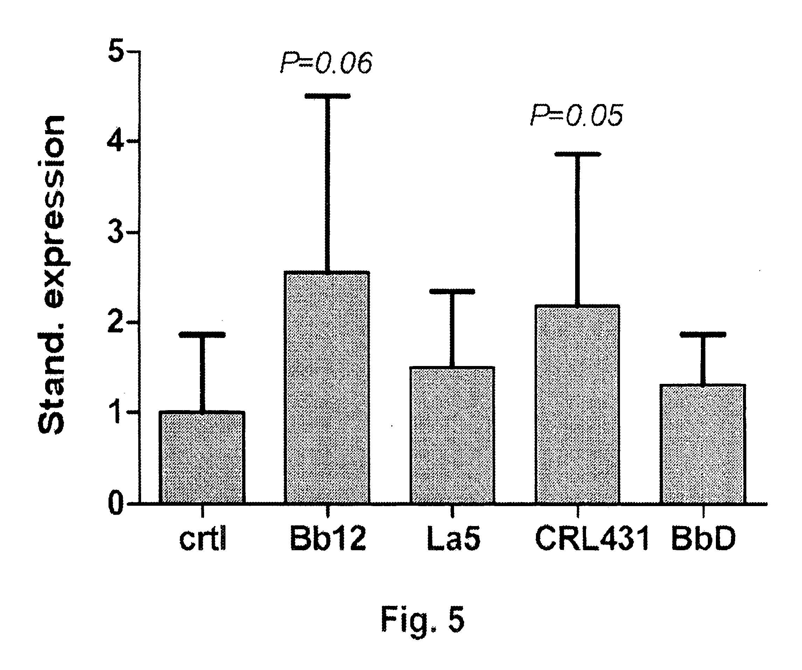

Figure 5. Expression of APOA4 in intestinal tissue. SCD-1 expression was quantified by Q-PCR on RNA extracted from mucosa samples. Each column represent average expression values and the error bar represent standard deviation. The average expression value of the control group (crtl) was set at 1.0 (n=6 piglets). Bb12: *Bifidobacterium animalis* subsp. *lactis* strain BB-12® (DSM15954) (n=7 piglets); La5: *Lactobacillus acidophilus* strain La5 (DSM13241) (n=6 piglets); CRL431: *Lactobacillus paracasei* subsp. *paracasei* strain CRL431, (ATCC 55544) (n=5 piglets).

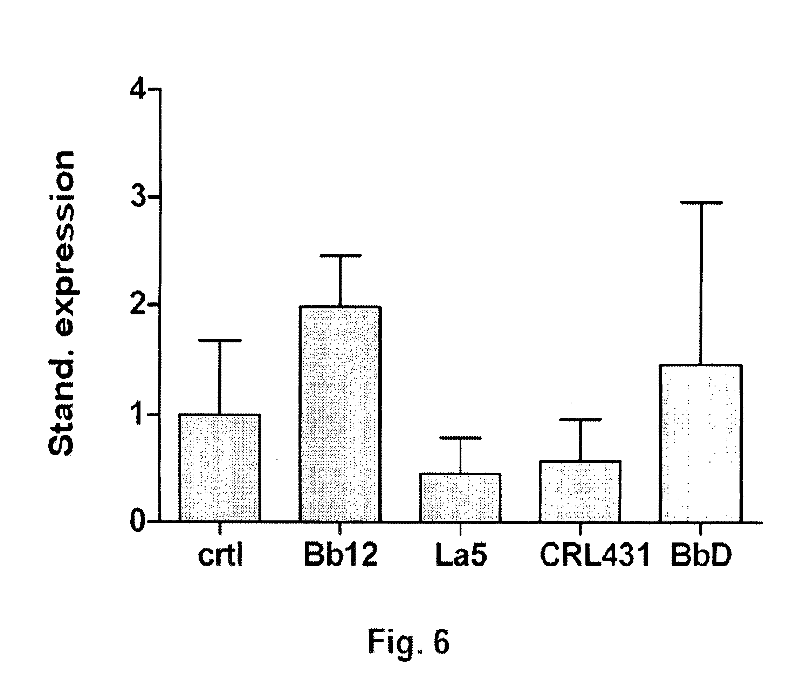

Figure 6. Expression of SCD-1 in skeletal muscle. SCD-1 expression was quantified by Q-PCR on RNA extracted from mucosa samples. Each column represent average expression values and the error bar represent standard deviation. The average expression value of the control group (crtl) was set at 1.0 (n=6 piglets). Bb12: *Bifidobacterium animalis* subsp. *lactis* strain BB-12® (DSM15954) (n=7 piglets); La5: *Lactobacillus acidophilus* strain La5 (DSM13241) (n=6 piglets); CRL431: *Lactobacillus paracasei* subsp. *paracasei* strain CRL431, (ATCC 55544) (n=5 piglets).

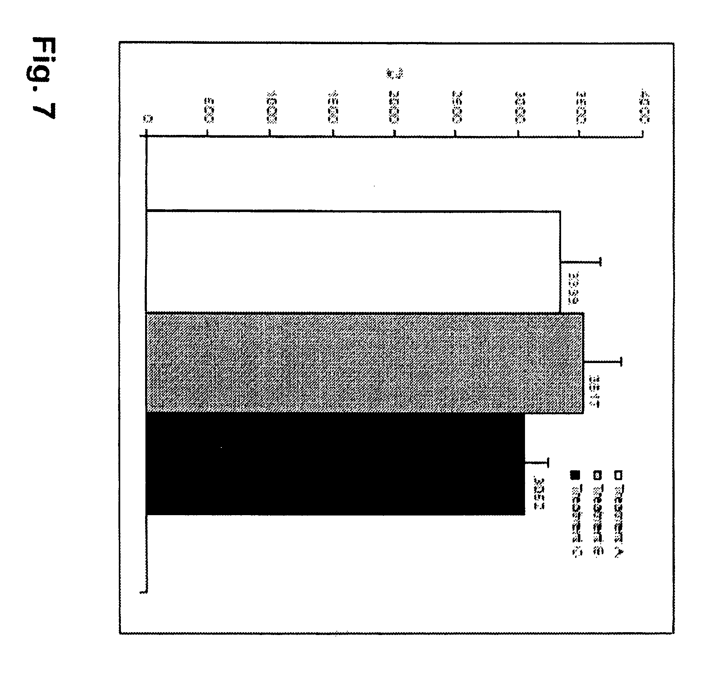

Figure 7. *Ad libitum* energy intake four hours after intake of capsule A (placebo), B (10⁹ CFU), or C (10¹⁰ CFU). N=21, mean ± SE..

### EXAMPLES

### Example 1: Probiotic strains up-regulate GCG expression in the ileum of pigs.

To investigate whether or not selected probiotic strains regulate ileal GCG expression in animals, young pigs were fed a standard diet including probiotic bacteria (i.e. *Bifidobacterium animalis* subsp. *lactis* strain BB-12® (DSM15954), *Lactobacillus acidophilus* strain La5 (DSM13241), and *Lactobacillus paracasei* subsp. *paracasei* strain CRL431, (ATCC 55544). The BB-12® strain is commercially available from Chr. Hansen A/S, 10-12 Boege Alle, DK-2970 Hoersholm, Denmark. Pigs fed with the same standard diet but not supplemented with probiotic bacteria served as control. Each group consisted of 8 piglets. At weaning at 4 weeks the animals were moved to pens where they were housed individually and assigned to the corresponding treatments for 14 days. Littermates were assigned to each of the treatments. The number of barrows and gilts in each treatment was the same. The pigs were fed twice daily, receiving an amount of feed corresponding to 4% of their body weight. The probiotics were given on top of the diet every morning.

Permission to carry out the experiment was granted from The Danish Plant Directorate and The Danish Ministry of Food, Agriculture and Fisheries.

After 14 days of treatment, the pigs were killed and tissues comprising 75% of the full length of the small intestine (i.e. the ileum or distal part of the small intestine) were sampled and snap-frozen in liquid nitrogen. Gene expression analysis on the distal ileum was performed by quantitative PCR analysis using primers specific for GCG. The quantitative PCR analysis was performed essentially as described by Kubista et al. (3524).

Primer sequences were,
GCG-F: 5'-TTC AGA ATA CAG AGG AGA AAT CCA-3'
GCG-R: 5'-AGT CAT CTG ATC TGG ATC ATC G-3'

As indicated in Figure 2, CRL-431 significantly up-regulates ileal GCG expression while the two other strains had no significant effect on GCG expression.

### Example 2: Effect of CRL431 on gut hormone release in isolated perfused pig intestine

One overnight fasted Danish LYY strain pig (XX kg) was anesthetized and an 80 cm section of the central ileum, including arterial and venous supply, was isolated. The segment was perfused with gassed (5% CO₂ in O₂) Krebs-Ringer bicarbonate perfusion buffer containing 0.1% human serum albumin; 5% dextran T-70; 7 mmol/L glucose; a mixture of amino acids (5 mmol/L); and 15-20% freshly washed bovine erythrocytes. A cyclooxygenase inhibitor was added to prevent generation of prostaglandins in the perfusion system. The tissue was perfused with the buffer at 24 mL/min. The gut lumen was perfused with perfusion buffer without erythrocytes at 3 mL/min. Perfusion pressure was recorded continuously and oxygen status and glucose levels were analyzed approx. every 30 min in venous effluent. The venous effluent was collected for 1 min periods. After centrifugation at 4 °C, the supernatants were divided into appropriate aliquots and stored in the freezer until analysis.

The experimental protocol consisted of perfusion of gut lumen with CRL431 (10⁸ CFU/mL) alone or in combination with an arterial bombesin stimuli (10⁻⁸ M).

Intact and total GLP-1, GLP-2, oxyntomodulin, and somatostatin were measured using previously described radioimmunoassays (14;34)25). PYY was measured using an ELISA kit from Linco (Millipore, MA, USA).

As indicated in figure 3, background levels of total GLP-1 secretion are at 75 ng/ml. Background levels of the released hormones are defined as the average of the measurements after the first bombesin stimulus, *i.e.* measurements obtained at time points 19 - 33 minutes. Perfusion of the intestinal lumen with CRL431 was carried out from time point 34 to 141 minutes. Perfusion with CRL431 gradually increases total GLP-1 levels to 215 ng/ml (defined as the average of the measurements after the first bombesin stimulus after bacterial infusion, *i.e.* measurements obtained at time points 176 - 205 minutes). The measurements correspond to a 287% increase in total GLP-1 levels.

An increase in the levels of intact, biologically active GLP-1 was also observed. Perfusion of the isolated pig intestine increased the levels of intact GLP-1 from 54 to 182 ng/ml, corresponding to an increase of 339%. Similarly, GLP-2 levels increased from 105 to 405 ng/ml, corresponding to an increase of 383%.

It is well-known that somatostatin inhibit the secretion of GLP-1 (36). In order to verify that the increased levels of intact and total GLP-1 and GLP-2 are not a result of decreased somatostatin expression from intestinal L-cells, we measured the levels of this hormone in the venous effluent. As shown in figure 3, the concentration of somatostatin increases during CRL431 perfusion from 40 to 93 ng/ml, corresponding to an increase of 237%. Thus, the increased expression of the proglucagon-derived hormones GLP-1 and GLP-2 is not due to a decrease in the expression of somatostatin.

Furthermore, as indicated in figure 3, background levels of PYY secretion are at 28 ng/ml. Perfusion with CRL431 gradually increases PYY levels to 65 ng/ml. The measurements correspond to a 228% increase in plasma PYY levels after CRL431 perfusion.

A surprising and highly interesting observation is seen after perfusion of the intestine with the *Lactobacillus paracasei* subsp. *paracasei* strain CRL431. After bacterial perfusion, the bombesin-induced release of total and intact GLP-1, GLP-2 and PYY is intensified with *peak values* of 624, 298, 639, and 148 ng/ml, respectively. This should be compared to *peak values* of total and intact GLP-1, GLP-2 and PYY of 281, 139, 316 and 79 ng/ml, respectively, during bacterial infusion.

In summary we have shown that CRL431 increase the levels of secreted GLP-1, GLP-2, and PYY, and that the bombesin-induced release GLP-1 GLP-2, and PYY is intensified (peak values).

### Example 3: Effect of CRL431 on PYY release in cultured human enteroendocrine cells.

Human enteroendocrine cells, NCI-H716, were cultured as previously described (37). CRL431 was cultivated in MRS (deMan, Rogosa and Sharpe, Difco, BD Diagnostics) and allowed to reach exponential growth phase. The bacteria were harvested by centrifugation and resuspeded after a single wash step in Krebs-Ringer buffer (Sigma, K4002) with 0.2% BSA (Sigma, A3294) to obtain the following concentrations: 1x10⁷, 5x10⁷, 7,5x10⁷ and 1x10⁸ cfu/ml.

Bacteria were incubated with the cell culture for two hours at 37°C. After incubation the supernatants were collected in test tubes containing dipeptidyl peptidase-4 (DPP-4) inhibitor (Millipore, MA, USA). PYY concentration of the cell culture supernatants was determined by ELISA (Linco, Millipore, MA, USA).

As indicated in figure 4 background levels of PYY were 19 pg/ml. Incubation of the NCI-H716 cell culture with increasing concentration of CRL431 triggers a dose-dependent release of PYY. At the highest concentration of CRL431, the PYY concentration reached a maximum of 215 pg/ml corresponding to an increase of 1131%.

### Example 4: Probiotic strain upregulate APOA4 expression in the pig intestine.

To investigate whether or not selected probiotic strains regulate ileal APOA4 expression in animals, young pigs were fed a standard diet including probiotic bacteria (i.e. *Bifidobacterium animalis* subsp. *lactis* strain BB-12® (DSM15954), *Lactobacillus acidophilus* strain La5 (DSM13241), and *Lactobacillus paracasei* subsp. *paracasei* strain CRL431, (ATCC 55544). Pigs fed with the same standard diet but not supplemented with probiotic bacteria served as control. Each group consisted of 8 piglets. At weaning at 4 weeks the animals were moved to pens where they were housed individually and assigned to the corresponding treatments for 14 days. Littermates were assigned to each of the treatments. The number of barrows and gilts in each treatment was the same. The pigs were fed twice daily, receiving an amount of feed corresponding to 4% of their body weight. The probiotics were given on top of the diet every morning.

Permission to carry out the experiment was granted from The Danish Plant Directorate and The Danish Ministry of Food, Agriculture and Fisheries.

After 14 days of treatment, the pigs were killed and tissues comprising 75% of the full length of the small intestine (i.e. the ileum or distal part of the small intestine) were sampled and snap-frozen in liquid nitrogen. Gene expression analysis on the distal ileum was performed by quantitative PCR analysis using primers specific for APOA4. The quantitative PCR analysis was performed essentially as described by Kubista et al. (35).

Primer sequences were,
APOA4-F: 5'- AAGGCCAAGATCGATCAGAA -3'
APOA4-R: 5'- GAGCTCCTCCGCATAGGG -3'

As indicated in Figure 5, CRL-431 up-regulates ileal APOA4 expression while the three other strains had no significant effect on APOA4 expression.

### Example 5: Probiotic strain down-regulate SCD-1 expression in the skeletal muscle of pigs.

To investigate whether or not selected probiotic strains regulate skeletal muscle SCD-1 expression in animals, young pigs were fed a standard diet including probiotic bacteria (i.e. *Bifidobacterium animalis* subsp. *lactis* strain BB-12® (DSM15954), *Lactobacillus acidophilus* strain La5 (DSM13241), and *Lactobacillus paracasei* subsp. *paracasei* strain CRL431, (ATCC 55544). Pigs fed with the same standard diet but not supplemented with probiotic bacteria served as control. Each group consisted of 8 piglets. At weaning at 4 weeks the animals were moved to pens where they were housed individually and assigned to the corresponding treatments for 14 days. Littermates were assigned to each of the treatments. The number of barrows and gilts in each treatment was the same. The pigs were fed twice daily, receiving an amount of feed corresponding to 4% of their body weight. The probiotics were given on top of the diet every morning.

Permission to carry out the experiment was granted from The Danish Plant Directorate and The Danish Ministry of Food, Agriculture and Fisheries.

After 14 days of treatment, the pigs were killed and tissues comprising skeletal muscle were sampled and snap-frozen in liquid nitrogen. Gene expression analysis on the distal ileum was performed by quantitative PCR analysis using primers specific for SCD-1. The quantitative PCR analysis was performed essentially as described by Kubista et al. (35).

Primer sequences were,
SCD1-F: 5'- GGGATACAGCTCCCCTCATAG -3'
SCD1-R: 5'- AGTTCCGATGTCTCAAAATGC -3'

As indicated in Figure 6, CRL-431 down-regulates skeletal muscle SCD-1 expression by approximately half the level of the non-treated pigs. This is comparable to the down-regulation observed for La-5. In contrast, Bb-12 and BbD (inactivated, dead Bb-12) appears to up-regulate muscle SCD-1 by 100% (for Bb-12) compared to non-treated pigs.

### Example 5: Effect of CRL431 on energy intake, and VAS scores.

The study was an intervention study with 22 healthy men and women, 20 to 45 years old, who would perform three single meal tests of approximately five hours duration. The study had a cross-over design where two different doses of probiotic bacteria or placebo were tested on appetite, ad libitum energy intake, and wellbeing. The participants were randomized to the order of the three different capsules e.g. placebo or 10⁹ CFU L. casei CRL431 or 10¹⁰ CFU L. casei CRL431. At the test days the participants would have either high dose probiotic capsule, low dose probiotic capsule or placebo capsule. These capsules were swallowed at the beginning of a standardized breakfast. Hereafter blood samples were taken and appetite vas registered until serving of an ad libitum lunch. To eliminate carry over effect of the capsules and for the safety of the participants there were a minimum of four weeks between the test days. The evening before the meal test the participants ate a standardized dinner (4.5 MJ) before 8.00 p.m. and were asked to fast hereafter except for a maximum of ½ L of water. On the day of the single meal test, the study participants arrived fasting at the Department at 8.00 a.m. by bus, train, metro or light/slow cycling. The participants were weighed in their underwear before resting and measurement of blood pressure. A venflon catheter was inserted in the right antecubital vein and at time 0 minutes a fasting blood sample was drawn, haemoglobin concentration was determined (> 7.5 mmol/l), and appetite was registered on visual analogue scale (VAS) questionnaires before the breakfast (2.5 MJ) and one of the three capsules was served. The participants had a maximum of 14 minutes to eat the breakfast. After the breakfast blood samples were drawn at time: 15, 30, 45, 60, 90, 120, 150, 180, 210 and 240 minutes after beginning of the breakfast adding up to 11 blood samples, 250 ml altogether.. Appetite was registered every half hour until ad libitum lunch was served four hours after the breakfast. After the lunch, appetite was registered for the last time and sensory quality of the meal was rated before the participants left the department.

Thirty-one subjects were screened and nine were excluded. A total of 22 subjects (eleven men and eleven women) were randomized and 21 completed all three visits in the study period. Mean age was 27.2 years and mean BMI was 23.6 kg/m2 and did not change significantly during the study period (p > 0.1). All participants had blood pressure within a normal range and had normal haemoglobin values, 7.5 - 10 mmol for women and 8 - 11 mmol for men, at every visit (Table 1).

**Table 1. Physical characteristics of participants at baseline.**

| | All (n = 21) | Men (n = 11) | Women (n = 10) |
|---|---|---|---|
| Age (y) | 27.2 ± 7.1¹ | 28.0 ± 7.9 | 26.3 ± 6.3 |
| Body composition Weight (kg) | 74.5 ± 12.8 | 84.6 ± 8.1 | 63.3 ± 5.2 |
| BMI (kg/m²) | 23.6 ± 1.4 | 24.2 ± 1.4 | 22.9 ± 1.1 |
| Blood pressure Systolic (mmHg) | 124.8 ± 10.8 | 132.4 ± 7.3 | 116.5 ± 7.4 |
| Diastolic (mmHg) | 78.6 ± 5.1 | 79.5 ± 6.3 | 77.6 ± 3.4 |
| Haemoglobin (mmol/L) | 8.8 ± 0.7 | 9.3 ± 0.4 | 8.3 ± 0.6 |

| | | | |
|---|---|---|---|
| Mean ± SD. | | | |

The subjective appetite sensation was expressed as VAS measured in mm. There were no differences in the participants' scores on satiety, fullness, hunger, and prospective food consumption when taking the different capsules adjusted for gender, BMI, and period (p > 0.1). In addition, composite appetite which is a sum of hunger, satiety, fullness and prospective intake scores did not differ between the groups (p > 0.1). There were no differences between the three kinds of treatments' effect on "desire for something sweet, fatty, salty and savoury" (p>0.1). Furthermore, the participants' well being was rated the same at all three treatments (p>0.1).

The VAS-scores were recalculated to give the area under the curve (AUC). As with the VASscores there were no differences in AUC for any of the VAS questions. However, when looking at the AUC results numerically the participants were most full and satisfied and felt less hunger and thought that they could eat less prospectively when having the capsule with high dose (HD) 10¹⁰ CFU L. casei CRL431 in comparison with placebo and low dose capsules (LD) 10⁹ CFU L. casei CRL431 (Table 2).

**Table 2 Area under the curve for appetite parameters and well being. All data are presented as mean ± SD (mm*min) (n=21).**

| **Treatment** | **Hunger** | **Full** | **Prospective food intake** | **Satiety** | **Well being** | **Composite appetite** |
|---|---|---|---|---|---|---|
| **Placebo** | 12,794±3,928 | 8,899±4,095 | 13,381±3,935 | 10,646±3,159 | 16,884±4,007 | 10,342±3,644 |
| **LD** | 12,948±3,798 | 8,634±3,945 | 13,424±3,699 | 10,243±3,545 | 16,577±4,223 | 10,126±3,577 |
| **HD** | 12,271±3,953 | 9,591±4,036 | 13,231±3,790 | 10,906±3,669 | 16,588±3,974 | 10,749±3,705 |

There was an overall effect of treatment on ad libitum energy intake at the lunch meal consumed four hours after taking the different capsules (p = 0.04). Posthoc pair wise comparison showed that the energy intake was 455 kJ or approximately 15 % lower after having consumed capsule 10¹⁰ CFU L. casei CRL431 in comparison to capsule 10⁹ CFU L. casei CRL431 (p = 0.03) (Figure 7). There was no difference in energy intakes between participants consuming placebo in comparison to capsules with 10¹⁰ CFU L. casei CRL431. These calculations were done after adjusting for gender, BMI, and period. Participants ate less at the ad libitum meal when having 10¹⁰CFU L. casei CRL431.

### Reference List

(1) Holst JJ. The physiology of glucagon-like peptide 1. Physiol Rev 2007;87(4):1409-39.
(2) Klonoff DC, Buse JB, Nielsen LL et al. Exenatide effects on diabetes, obesity, cardiovascular risk factors and hepatic biomarkers in patients with type 2 diabetes treated for at least 3 years. Curr Med Res Opin 2008;24(1):275-86.
(3) Verdich C, Flint A, Gutzwiller JP et al. A meta-analysis of the effect of glucagon-like peptide-1 (7-36) amide on ad libitum energy intake in humans. J Clin Endocrinol Metab 2001;86(9):4382-9.
(4) Tolessa T, Gutniak M, Holst JJ et al. Glucagon-like peptide-1 retards gastric emptying and small bowel transit in the rat: effect mediated through central or enteric nervous mechanisms. Dig Dis Sci 1998;43(10):2284-90.
(5) Kreymann B, Ghatei MA, Burnet P et al. Characterization of glucagon-like peptide-1-(7-36)amide in the hypothalamus. Brain Res 1989;502(2):325-31.
(6) Larsen PJ, Tang-Christensen M, Holst JJ et al. Distribution of glucagon-like peptide-1 and other preproglucagon-derived peptides in the rat hypothalamus and brainstem. Neuroscience 1997;77(1):257-70.
(7) Wei Y, Mojsov S. Tissue-specific expression of the human receptor for glucagon-like peptide-I: brain, heart and pancreatic forms have the same deduced amino acid sequences. FEBS Lett 1995;358(3):219-24.
(8) Shughrue PJ, Lane MV, Merchenthaler I. Glucagon-like peptide-1 receptor (GLP1-R) mRNA in the rat hypothalamus. Endocrinology 1996;137(11):5159-62.
(9) Burrin DG, Petersen Y, Stoll B et al. Glucagon-like peptide 2: a nutrient-responsive gut growth factor. J Nutr 2001;131(3):709-12.
(10) Tang-Christensen M, Larsen PJ, Thulesen J et al. The proglucagon-derived peptide, glucagon-like peptide-2, is a neurotransmitter involved in the regulation of food intake. Nat Med 2000;6(7):802-7.
(11) Benjamin MA, McKay DM, Yang PC et al. Glucagon-like peptide-2 enhances intestinal epithelial barrier function of both transcellular and paracellular pathways in the mouse. Gut 2000;47(1):112-9.
(12) Cani PD, Amar J, Iglesias MA et al. Metabolic endotoxemia initiates obesity and insulin resistance. Diabetes 2007;56:1761-72.
(13) Parlevliet ET, Heijboer AC, Schroder-van der Elst JP et al. Oxyntomodulin ameliorates glucose intolerance in mice fed a high-fat diet. Am J Physiol Endocrinol Metab 2008;294(1): E142-E147.
(14) Schjoldager BT, Baldissera FG, Mortensen PE et al. Oxyntomodulin: a potential hormone from the distal gut. Pharmacokinetics and effects on gastric acid and insulin secretion in man. Eur J Clin Invest 1988;18(5):499-503.
(15) Cohen MA, Ellis SM, Le Roux CW et al. Oxyntomodulin suppresses appetite and reduces food intake in humans. J Clin Endocrinol Metab 2003;88(10):4696-701.
(16) Wynne K, Park AJ, Small CJ et al. Oxyntomodulin increases energy expenditure in addition to decreasing energy intake in overweight and obese humans: a randomised controlled trial. Int J Obes (Lond) 2006;30(12):1729-36.
(17) Dakin CL, Small CJ, Batterham RL et al. Peripheral oxyntomodulin reduces food intake and body weight gain in rats. Endocrinology 2004;145(6):2687-95.
(18) Wynne K, Park AJ, Small CJ et al. Subcutaneous oxyntomodulin reduces body weight in overweight and obese subjects: a double-blind, randomized, controlled trial. Diabetes 2005;54(8):2390-5.
(19) Wallis K, Walters JR, Forbes A. Review article: glucagon-like peptide 2--current applications and future directions. Aliment Pharmacol Ther 2007;25(4):365-72.
(20) Tomita R, Igarashi S, Tanjoh K et al. Role of recombinant human glicentin in the normal human jejunum: an in vitro study. Hepatogastroenterology 2005;52(65):1459-62.
(21) Ayachi SE, Borie F, Magous R et al. Contraction induced by glicentin on smooth muscle cells from the human colon is abolished by exendin (9-39). Neurogastroenterol Motil 2005;17(2):302-9.
(22) Nygaard R, Nielbo S, Schwartz TW et al. The PP-Fold Solution Structure of Human Polypeptide YY and Human PYY3-36 As Determined by NMRΓÇá,ΓÇi Biochemistry 2006;45(27):8350-7.
(23) Murphy KG, Bloom SR. Gut hormones and the regulation of energy homeostasis. Nature 2006;444(7121):854-9.
(24) Taylor IL. Distribution and release of peptide YY in dog measured by specific radioimmunoassay. Gastroenterology 1985;88(3):731-7.
(22) Zhang J, Tokui Y, Yamagata K et al. Continuous stimulation of human glucagon-like peptide-1 (7-36) amide in a mouse model (NOD) delays onset of autoimmune type 1 diabetes. Diabetologia 2007;50(9):1900-9.
(23) Blandino-Rosano M, Perez-Arana G, Mellado-Gil J et al. Anti-proliferative Effect of Pro-inflammatory Cytokines in Cultured Beta Cells is Associated with Erk1/2 Pathway Inhibition: Protective Role of GLP-1. J Mol Endocrinol 2008.
(24) Kubista M, Andrade JM, Bengtsson M et al. The real-time polymerase chain reaction. Mol Aspects Med 2006;27(2-3):95-125.
(25) Liu CD, Aloia T, Adrian TE et al. Peptide YY: a potential proabsorptive hormone for the treatment of malabsorptive disorders. Am Surg 1996;62(3):232-6.
(26) Batterham RL, Cowley MA, Small CJ et al. Gut hormone PYY(3-36) physiologically inhibits food intake. Nature 2002;418(6898):650-4.
(27) Kalogeris TJ, Rodriguez MD, Tso P. Control of Synthesis and Secretion of Intestinal Apolipoprotein A-IV by Lipid. J Nutr 1997;127(3):537S.
(28) Shen L, Tso P, Woods SC et al. Hypothalamic Apolipoprotein A-IV Is Regulated by Leptin. Endocrinology 2007;148(6):2681-9.
(29) Tso P, Liu M. Apolipoprotein A-IV, food intake, and obesity. Physiology & Behavior 2004;83(4):631-43.
(30) Woods SC. Dietary synergies in appetite control: distal gastrointestinal tract. Obesity (Silver Spring) 2006;14 Suppl 4:171S-8S.
(31) Hulver MW, Berggren JR, Carper MJ et al. Elevated stearoyl-CoA desaturase-1 expression in skeletal muscle contributes to abnormal fatty acid partitioning in obese humans. Cell Metab 2005;2(4):251-61.
(32) Zhang J, Tokui Y, Yamagata K et al. Continuous stimulation of human glucagon-like peptide-1 (7-36) amide in a mouse model (NOD) delays onset of autoimmune type 1 diabetes. Diabetologia 2007;50(9):1900-9.
(33) Blandino-Rosano M, Perez-Arana G, Mellado-Gil J et al. Anti-proliferative Effect of Pro-inflammatory Cytokines in Cultured Beta Cells is Associated with Erk1/2 Pathway Inhibition: Protective Role of GLP-1. J Mol Endocrinol 2008.
(34) Orskov C, Holst JJ, Knuhtsen S et al. Glucagon-like peptides GLP-1 and GLP-2, predicted products of the glucagon gene, are secreted separately from pig small intestine but not pancreas. Endocrinology 1986;119(4):1467-75.
(35) Kubista M, Andrade JM, Bengtsson M et al. The real-time polymerase chain reaction. Mol Aspects Med 2006;27(2-3):95-125.
(36) Hansen L, Hartmann B, Bisgaard T et al. Somatostatin restrains the secretion of glucagon-like peptide-1 and -2 from isolated perfused porcine ileum. Am J Physiol Endocrinol Metab 2000;278(6):E1010-E1018.
(37) Reimer RA, Darimont C, Gremlich S et al. A human cellular model for studying the regulation of glucagon-like peptide-1 secretion. Endocrinology 2001;142(10):4522-8.

NIH (1998): Blount, A. B. S. et al.: CLINICAL GUIDELINES ON THE IDENTIFICATION, EVALUATION, AND TREATMENT OF OVERWEIGHT AND OBESITY IN ADULTS The Evidence Report. NIH PUBLICATION NO. 98-4083. SEPTEMBER 1998. NATIONAL INSTITUTES OF HEALTH.
Neovius et al (2009) Combined effects of overweight and smoking in late adolescence on subsequent mortality: nationwide cohort study. British Medical Journal) 338, b496.
FAO/WHO (2001) Joint FAO/WHO Expert Consultation on Evaluation of Health and Nutritional Properties of Probiotics in Food Including Powder Milk with Live Lactic Acid Bacteria, October 2001 (ftp://ftp.fao.org/docrep/fao/meeting/009/y6398e.pdf)

## Claims

1. A composition comprising *Lactobacillus paracasei* subsp. *paracasei* CRL431 having accession number ATCC 55544 for use in the prevention and/or treatment of obesity.

2. A composition comprising *Lactobacillus paracasei* subsp. *paracasei* CRL431 having accession number ATCC 55544 for use in the prevention and/or treatment of type 2 diabetes mellitus or polycystic ovary syndrome (PCOS).

3. The composition for use according to claim 1 or 2, wherein the *Lactobacillus paracasei* subsp. *paracasei* CRL431 having accession number ATCC 55544 is combined with at least one prebiotic.

4. The composition for use according to claim 3, wherein the at least one prebiotic is selected from the group consisting of: inulin, a transgalacto-oligosaccharide, palatinoseoligosaccharide, soybean oligosaccharide, gentiooligosaccharide, xylooligomers, nondegradable starch, lactosaccharose; lactulose, lactitol, maltitol, FOS (fructo-oligosaccharides), GOS (galacto-oligosaccharides), and polydextrose.

5. The composition for use according to any of the preceding claims, wherein the strain of *Lactobacillus paracasei* subsp. *paracasei* CRL431 having accession number ATCC 55544 increases the level of GLP-1.

6. A cosmetic method for reducing body weight in a non-obese, non-overweight subject having a Body Mass Index (BMI) less than 25, said method comprising providing a composition comprising *Lactobacillus paracasei* subsp. *paracasei* CRL431 having accession number ATCC 55544.

7. A cosmetic method for reducing body weight in a non-obese subject, said method comprising providing a composition comprising *Lactobacillus paracasei* subsp. *paracasei* CRL431 having accession number ATCC 55544.

## Patentansprüche

1. Zusammensetzung mit *Lactobacillus paracasei* subsp. paracasei CRL431 mit Zugangsnummer ATCC 55544 zum Einsatz bei der Vorbeugung und/oder Behandlung von Fettleibigkeit.

2. Zusammensetzung mit *Lactobacillus paracasei* subsp. paracasei CRL431 mit Zugangsnummer ATCC 55544 zum Einsatz bei der Vorbeugung und/oder Behandlung von Typ-2-Diabetes oder polyzystischem Ovarialsyndrom (PCOS).

3. Zusammensetzung nach Anspruch 1 oder 2, wobei der *Lactobacillus paracasei* subsp. *paracasei* CRL431 mit Zugangsnummer ATCC 55544 mit mindestens einem Präbiotikum kombiniert wird.

4. Zusammensetzung nach Anspruch 3, wobei das mindestens eine Präbiotikum aus folgender Gruppe ausgewählt ist: Inulin, ein Transgalactooligosaccharid, Palatinose-Oligosaccharid, Sojabohnenoligosaccharid, Gentiooligosaccharid, Xylooligomere, nicht abbaubare Stärke, Lactosaccharose; Lactulose, Lactitol, Maltit, FOS (Fructooligosaccharide), GOS (Galacto-Oligosaccharide) und Polydextrose.

5. Zusammensetzung nach einem der vorhergehenden Patentansprüche, wobei der Stamm von *Lactobacillus paracasei* subsp. *paracasei* CRL431 mit Zugangsnummer ATCC 55544 den GLP-1-Spiegel erhöht.

6. Kosmetische Methode zur Verringerung des Körpergewichts einer nicht fettleibigen, nicht übergewichtigen Person mit einem Body-Mass-Index (BMI) unter 25, wobei das Verfahren eine Zusammensetzung mit *Lactobacillus paracasei* subsp. *paracasei* CRL431 mit Zugangsnummer ATCC 55544 umfasst.

7. Kosmetische Methode zur Verringerung des Körpergewichts einer nicht fettleibigen Person, wobei das Verfahren eine Zusammensetzung mit *Lactobacillus paracasei* subsp. *paracasei* CRL431 mit Zugangsnummer ATCC 55544 umfasst.

## Revendications

1. Composition comprenant une *Lactobacillus paracasei* sous-espèce *paracasei* CRL431 ayant le numéro d'accès ATCC 55544 destinée à être utilisée pour la prévention et/ou le traitement de l'obésité.

2. Composition comprenant une *Lactobacillus paracasei* sous-espèce *paracasei* CRL431 ayant le numéro d'accès ATCC 55544 destinée à être utilisée pour la prévention et/ou le traitement du diabète mellitus de type 2 ou du syndrome des ovaires polykystiques (SOPK).

3. Composition pour une utilisation selon la revendication 1 ou 2, où la *Lactobacillus paracasei* sous-espèce *paracasei* CRL431 ayant le numéro d'accès ATCC 55544 est combinée avec au moins un prébiotique.

4. Composition pour une utilisation selon la revendication 3, où le au moins un prébiotique est sélectionné au sein du groupe consistant en: inuline, un transgalacto-oligosaccharide, oligosaccharide de palatinose, oligosaccharide de soja, gentio-oligosaccharide, xylo-oligomères, amidon non dégradable, lactosaccharose; lactulose, lactitol, maltitol, FOS (fructo-oligosaccharides), GOS (galacto-oligosaccharides) et polydextrose.

5. Composition pour une utilisation selon l'une quelconque des revendications précédentes, où la souche de *Lactobacillus paracasei* sous-espèce *paracasei* CRL431 ayant le numéro d'accès ATCC 55544 augmente le niveau de GLP-1.

6. Procédé cosmétique de réduction du poids corporel chez un sujet non obèse sans surcharge pondérale ayant un indice de masse corporelle (IMC) inférieur à 25, ledit procédé comprenant une composition comprenant le *Lactobacillus paracasei* sous-espèce *paracasei* CRL431 ayant le numéro d'accès ATCC 55544.

7. Procédé cosmétique de réduction du poids corporel chez un sujet non obèse, ledit procédé comprenant une composition comprenant la *Lactobacillus paracasei* sous-espèce *paracasei* CRL431 ayant le numéro d'accès ATCC 55544.
